(19) **Europäisches Patentamt / European Patent Office / Office européen des brevets**

(11) **EP 3 053 537 A1**

(12) **EUROPEAN PATENT APPLICATION**

(43) Date of publication:
**10.08.2016 Bulletin 2016/32**

(51) Int Cl.:
**A61B 18/14** (2006.01) **A61B 18/12** (2006.01)
**A61B 18/00** (2006.01)

(21) Application number: **16156267.3**

(22) Date of filing: **16.11.2010**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.11.2009 US 619462**

(62) Document number(s) of the earlier application(s) in accordance with Art. 76 EPC:
**10191321.8 / 2 322 111**

(71) Applicant: **Covidien LP**
**Mansfield, MA 02048 (US)**

(72) Inventor: **LADTKOW, Casey M**
**Westminister, CO Colorado CO 80031 (US)**

(74) Representative: **Pratt, Richard Wilson**
**Maschio & Soames IP Limited**
**30 Carlton Crescent**
**Southampton SO15 2EW (GB)**

Remarks:
This application was filed on 18-02-2016 as a divisional application to the application mentioned under INID code 62.

(54) **MULTI-PHASE ELECTRODE**

(57) An electrosurgical system (1), comprises an electrosurgical generator (20) configured to generate a phased electrosurgical voltage and an electrosurgical instrument (100, 200). The instrument includes a core (102, 202) having an elongated body portion (103, 203) and at least one channel defined therein; a plurality of electrodes (110a, b, c; 210 a, b, c) disposed about the core, each of the plurality of the electrodes coupled to the electrosurgical generator such that the phased electrosurgical voltage supplied to the plurality of electrodes generates a potential difference between at least two of the plurality of the electrodes; and a plurality of spring conductors (116a, 116b, 216a) disposed within the at least one channel and configured to couple each of the plurality of electrodes to the electrosurgical generator.

FIG. 3

## Description

## BACKGROUND

*Technical Field*

The present disclosure relates to electrosurgical apparatuses, systems and methods. More particularly, the present disclosure is directed to an electrosurgical instrument having a plurality of electrodes that operates at different phases.

*Background of Related Art*

Energy-based tissue treatment is well known in the art. Various types of energy (e.g., electrical, ultrasonic, microwave, cryogenic, heat, laser, etc.) are applied to tissue to achieve a desired result. Electrosurgery involves application of high radio frequency electrical current to a surgical site to cut, ablate, coagulate or seal tissue.

In monopolar electrosurgery, the active electrode is typically a part of the surgical instrument held by the surgeon that is applied to the tissue to be treated. A patient return electrode is placed remotely from the active electrode to carry the current back to the generator and safely disperse current applied by the active electrode. The return electrodes usually have a large patient contact surface area to minimize heating at that site. Heating is caused by high current densities which directly depend on the surface area. A larger surface contact area results in lower localized heat intensity. Return electrodes are typically sized based on assumptions of the maximum current utilized during a particular surgical procedure and the duty cycle (i.e., the percentage of time the generator is on).

## SUMMARY

According to one aspect of the present disclosure, an electrosurgical instrument is disclosed. The instrument includes a core including an elongated body portion, a plurality of electrodes having a tubular shape and disposed about the core and one or more dielectric spacers disposed between each of the plurality of the electrodes. The plurality of the electrodes is coupled to an electrosurgical generator configured to supply phased electrosurgical voltage to one or more of the plurality of electrodes to generate a potential difference between two or more of the plurality of the electrodes.

According to another embodiment an electrosurgical system is disclosed. The system includes an electrosurgical instrument having a core including an elongated body portion, a plurality of electrodes having a tubular shape and disposed about the core and one or more dielectric spacers disposed between each of the plurality of the electrodes. The system also includes an electrosurgical generator coupled to each of the plurality of the electrodes and configured to supply phased electrosurgical voltage to one or more of the plurality of electrodes to generate a potential difference between two or more of the plurality of the electrodes.

According to a further embodiment of the present disclosure an electrosurgical instrument is disclosed. The instrument includes a core having an elongated body portion and a plurality of electrodes having a tubular shape and disposed about the core. The instrument also includes one or more dielectric spacers disposed between each of the plurality of the electrodes and a plurality of conductors configured to couple each of the plurality of electrodes to an electrosurgical generator configured to supply phased electrosurgical voltage to at least one of the plurality of electrodes to generate a potential difference between at least two of the plurality of the electrodes.

## BRIEF DESCRIPTION OF THE DRAWINGS

Various embodiments of the present disclosure are described herein with reference to the drawings wherein:

Fig. 1 is a schematic diagram of an electrosurgical system according to one embodiment of the present disclosure;
Fig. 2 is a schematic block diagram of the electrosurgical generator of Fig. 1 according to an embodiment of the present disclosure;
Fig. 3 is perspective, cross-sectional view of an electrode assembly according to one embodiment of the present disclosure;
Fig. 4 is a side, cross-sectional view of an electrode assembly of Fig. 3 according to the present disclosure;
Fig. 5 is a side, cross-sectional view of a conductor according to one embodiment of the present disclosure;
Fig. 6 is perspective, cross-sectional view of an electrode assembly according to another embodiment of the present disclosure;
Fig. 7 is a side, cross-sectional view of an electrode assembly of Fig. 6 according to the present disclosure; and

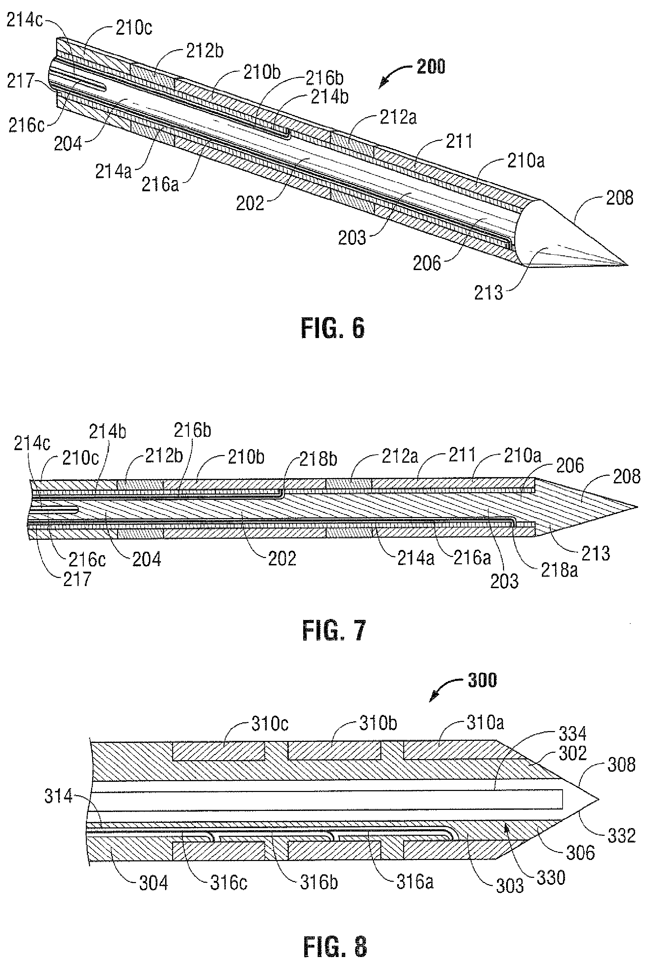

Fig. 7 is a side, cross-sectional view of an electrode assembly according to a further embodiment of the present disclosure.

## DETAILED DESCRIPTION

Particular embodiments of the present disclosure are described hereinbelow with reference to the accompanying drawings. In the following description, well-known functions or constructions are not described in detail to avoid obscuring the present disclosure in unnecessary detail.

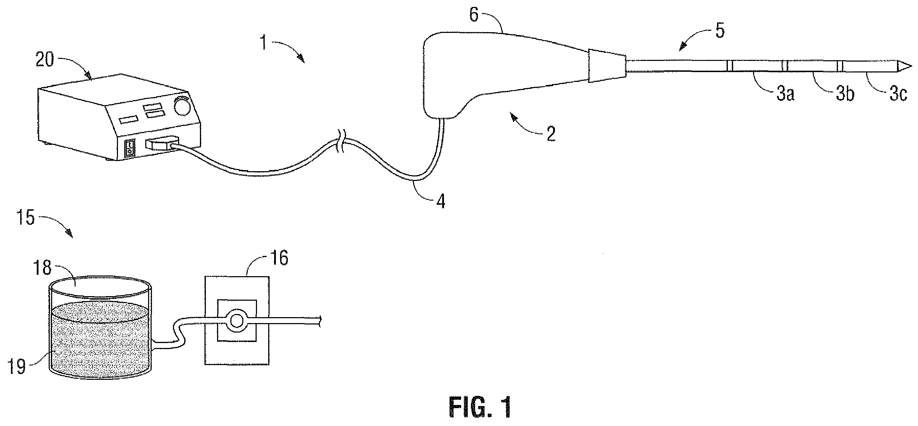

Fig. 1 is a schematic illustration of a multipolar electrosurgical system 1 according to one embodiment of the present disclosure. The system includes one or more multipolar electrosurgical instruments 2 having a housing 6 with an electrode assembly 5 coupled thereto and extending distally therefrom. The electrode assembly 5 includes one or more electrodes 3a, 3b, 3c, etc. (e.g., electrosurgical cutting probe, ablation electrode(s), etc.) for treating tissue of a patient. The instrument 2 is coupled to a generator 20 via a cable 4 that encloses a plurality of supply and return lines. More specifically, the electrosurgical RF energy is supplied to the instrument 2 is via the generator 20, allowing the instrument 2 to coagulate, ablate and/or otherwise treat tissue. The energy is also returned to the generator 20 through the electrodes 3a, 3b, 3c, etc.

The instrument 2 may also be coupled to a coolant system 15 that includes a supply pump 16 and to a supply tank 18. The supply pump 16 may be a peristaltic pump or any other suitable type of pump. The supply tank 18 stores the coolant fluid 19 and, in one embodiment, may maintain the fluid at a predetermined temperature. In another embodiment, the coolant fluid 19 may be a gas and/or a mixture of fluid and gas. The coolant system 15 is configured to circulate the coolant fluid 19 through the electrode assembly 5, thereby cooling the electrodes as discussed in more detail below.

The generator 20 is configured to operate in a variety of modes. In one embodiment, the generator 20 may operate in the following modes: cut, blend, division with hemostasis, fulgurate and spray. Each of the modes operates based on a preprogrammed power curve that dictates how much power is outputted by the generator 20 at varying impedance ranges of the load (e.g., tissue). Each of the power curves includes a constant power, constant voltage and constant current ranges that are defined by the user-selected power setting and the measured minimum impedance of the load.

In a cut mode, the generator 20 supplies a continuous sine wave at a predetermined frequency (e.g., 472 kHz) having a crest factor of 1.5 or less in the impedance range of 100Ω to 2,000Ω. The cut mode power curve may include three regions: constant current into low impedance, constant power into medium impedance and constant voltage into high impedance. In the blend mode, the generator supplies bursts of a sine wave at the predetermined frequency, with the bursts reoccurring at a first predetermined rate (e.g., about 26.21 kHz). In one embodiment, the duty cycle of the bursts may be about 50%. The crest factor of one period of the sine wave may be less than 1.5. The crest factor of the burst may be about 2.7.

The division with hemostasis mode includes bursts of sine waves at a predetermined frequency (e.g., 472 kHz) reoccurring at a second predetermined rate (e.g., about 28.3 kHz). The duty cycle of the bursts may be 25%. The crest factor of one burst may be 4.3 across an impedance range of 100Ω to 2,000Ω. The fulgurate mode includes bursts of sine waves at a predetermined frequency (e.g., 472 kHz) reoccurring at a third predetermined rate (e.g., about 30.66 kHz). The duty cycle of the bursts may be 6.5% and the crest factor of one burst is 5.55 across an impedance range of 100Ω to 2,000Ω. The spray mode will be bursts of sine waves at a predetermined frequency (e.g., 472 kHz) reoccurring at a fourth predetermined rate (e.g., about 21.7 kHz). The duty cycle of the bursts may be 4.6% and the crest factor of one burst may be 6.6 across the impedance range of 100Ω to 2,000Ω.

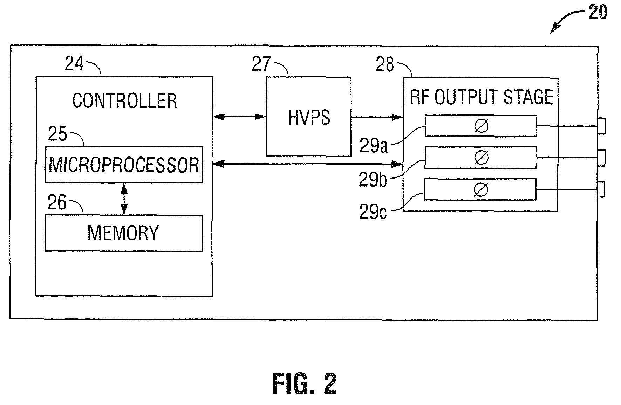

Fig. 2 shows a schematic block diagram of the generator 20 having a controller 24, a high voltage DC power supply 27 ("HVPS") and an RF output stage 28. The HVPS 27 is connected to an AC source (e.g., electrical wall outlet) and provides high voltage DC power to an RF output stage 28, which then converts high voltage DC power into RF energy and delivers the RF energy to the instrument 2. In particular, the RF output stage 28 generates sinusoidal waveforms of high RF energy. The RF output stage 28 is configured to operate in a plurality of modes, during which the generator 20 outputs corresponding waveforms having specific duty cycles, peak voltages, crest factors, etc.

The controller 24 includes a microprocessor 25 operably connected to a memory 26, which may be volatile type memory (e.g., RAM) and/or non-volatile type memory (e.g., flash media, disk media, etc.). The microprocessor 25 includes an output port that is operably connected to the HVPS 27 and/or RF output stage 28 allowing the microprocessor 25 to control the output of the generator 20 according to either open and/or closed control loop schemes. Those skilled in the art will appreciate that the microprocessor 25 may be substituted by any logic processor (e.g., control circuit) adapted to perform the calculations discussed herein.

A closed loop control scheme is a feedback control loop, in which a plurality of sensors measure a variety of tissue and energy properties (e.g., tissue impedance, tissue temperature, output current and/or voltage, etc.), and provide feedback to the controller 24. Such sensors are within the purview of those skilled in the art. The controller 24 then signals the HVPS 27 and/or RF output stage 28, which then adjusts the DC and/or RF power supply, respectively. The controller 24 also receives input signals from the input controls of the generator 20 and/or the instrument 2a. The controller 24 utilizes the input signals to adjust power outputted by the generator 20 and/or performs other control functions thereon.

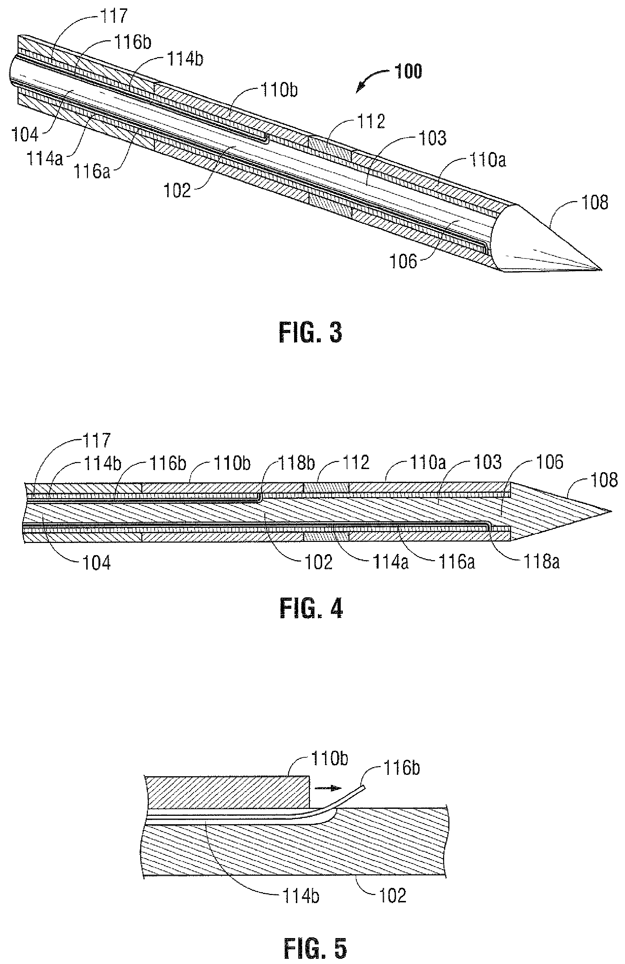

Figs. 3 and 4 show an electrode assembly 100 according to an embodiment of the present disclosure. The electrode assembly 100 includes a core 102 having an elongated body portion 103. The body portion 103 includes a proximal end 104 coupled to the housing 6 (Fig. 1) and a distal end 106 having a tip 108. The tip 108 may be formed integrally with the body portion 103. The tip 108 includes a tapered portion terminating in a sharp tip to allow for insertion into tissue with minimal resistance. In those cases where the energy applicator is inserted into a pre-existing opening, the tip 108 may be rounded or flat.

The electrode assembly 100 also includes two or more electrodes 110a and 110b. The electrodes 110a and 110b have a substantially cylindrical tubular shape and are disposed about the core 102. The electrodes 110a and 110b are separated by a dielectric spacer 112 that also has a cylindrical tubular shape. The electrodes 110a and 110b may be formed from any suitable medical grade conductor suitable for contacting tissue (e.g.. stainless steel).

In one embodiment, the dielectric spacer 112 may be integrally formed with the core 102. The core 102, the dielectric spacer 112 and the tip 108 may be formed from a suitable polymeric material, which may include, for example, thermoplastics including reinforced or unreinforced polymers, e.g., polyamide (nylon) or polyaramid (e.g., KEVLAR® manufactured by E. I. du Pont de Nemours and Company of Wilmington, Delaware, United States), or any suitable polymeric composite, e.g., polymers filled with carbon particles, silica, conductive particles such as metal particles or conductive polymers, or combinations thereof.

The core 102 includes one or more channels 114a and 114b defined therein. In one embodiment, a single channel (e.g., channel 114a) may be utilized to route the plurality of conductors 116a and 116b to the electrodes 110a and 110b. In this embodiment, the conductors 116a and 116b include an insulating sheath to prevent short-circuiting. In another embodiment, the channels 114a and 114b house one or more conductors 116a and 116b, respectively. The conductors 116a and 116b may be encased in an insulating sheath (not shown) and coupled to the electrodes 110a and 110b, respectively. The channels 114a and 114b are disposed apart (e.g., radially) within the core 102 and extend up to the respective electrodes 110a and 110b. The dielectric structure of the core 102 obviates the need for providing an insulating sheath about the conductors 116a and 116b since the conductors 116a and 116b are separated by the core 102 and only contact the corresponding electrodes 110a and 110b. The core 102 may also be formed from conductive materials (e.g., metal) and may include a sheath 117 disposed over the outer surface thereof to insulate the core 102 from the electrodes 110a and 110b.

In one embodiment, the channels 114a and 114b may be disposed on an outer surface of the core 102. As shown in Fig. 4, the core 102 may include a sheath 117 disposed over the body portion 103 to insulate the conductors 116a and 116b. The sheath 117 includes openings 118a and 118b to provide for coupling of the conductors 116a and 116b to the electrodes 114a and 114b (Fig. 4).

In another embodiment, as shown in Fig. 5, the conductors 116a and 116b may be formed from a spring member (e.g., hardened spring steel) to ensure that the conductors 116a and 116b are pushed into the corresponding channels 114a and 114b as the electrodes 110a and 110b are pushed into place. This configuration also secured the conductors 116a and 116b within the channels 114a and 114b.

With reference to Fig. 2, in one embodiment, the generator 20 is a multi-phase radio-frequency energy generator that can supply electrosurgical energy across each voltage line corresponding to a specific phase. Each of the electrodes 110a and 110b is connected to a voltage line of the generator 20. The generator includes a plurality of phase-shifting circuits 29a, 29b, 29c, etc., each of which is coupled to each of the electrodes 110a and 110b (with one of the phase shifting circuits remaining unused). The output voltages from these phase-shifting circuits 29a, 29b, 29c have substantially the same amplitudes, however, the phases of the voltages are shifted relative to each other. The phase-shifting circuits 29a, 29b, 29c shift the phases of the voltage line based on the number of electrodes 110a and 110b connected thereto. In other words, the phase shift may be defined by the following formula (1):

$$(1) \quad \phi = \frac{360°}{n}$$

In formula (1), n is the number of groups of electrodes. More specifically, a group of electrodes may include one or more electrodes. The electrode assembly 100 may include a total of four electrodes, with two groups of two electrodes. In this instance, each of the groups of the electrodes is coupled to one of the two phase-shifting circuits 29a, 29b, 29c. Based on the formula (1), the phase shift between the two groups of electrodes is 180°. In the embodiment of Figs. 3 and 4, where there are two electrodes 110a and 110b, each electrodes forms its own group, therefore the voltages supplied thereto are shifted by 180°.

In use, the generator 20 supplies phased electrosurgical voltage to each of the electrodes 110a and 110b. In other words, the phase-shifting circuits 29a, 29b, 29c energize one of the electrodes 110a and 110b, while the non-energized electrodes 110a and 110b act as a return electrode. This flow of energy between the electrodes 110a and 110b is due to the phase shift therebetween, which provides for a sufficient potential difference therebetween. The electrodes 110a and 110b shift in their roles as active and return electrodes due to the phase-shifted application of voltage.

Figs. 6 and 7 show another embodiment of an electrosurgical electrode assembly 200 according to another embodiment of the present disclosure. The electrode assembly 200 includes a core 202 having an elongated cylindrical body portion 203. The body portion 203 includes a proximal end 204 coupled to the housing 6 (Fig. 1) and a distal end 206.

The electrode assembly 200 also includes three or more electrodes 210a, 210b, 210c. The electrodes 210a, 210b, 210c may be formed from any suitable medical grade conductor suitable for contacting tissue (e.g., stainless steel). The electrodes 210b and 210c have a substantially cylindrical tubular shape and are disposed about the core 202. The electrode 210a includes a proximal portion 211 that also has a substantially cylindrical tubular shape and is disposed about the core 202 and a distal portion 213 that includes a tip 208. The tip 208 includes a tapered portion terminating in a sharp tip to allow for insertion into tissue with minimal resistance. In those cases where the energy applicator is inserted into a pre-existing opening, the tip 208 may be rounded or flat.

The electrodes 210a, 210b, 210c are separated by dielectric spacers 212a and 212b, with the dielectric spacer 212a disposed between the electrodes 210a and 210b and the dielectric spacer 212b disposed between the electrodes 210b and 210c. The dielectric spacers 212a and 212b also have a cylindrical tubular shape. In one embodiment, the dielectric spacers 212a and 212b may be integrally formed with the core 202.

The core 202 includes one or more channels 214a, 214b, 214c defined therein. In one embodiment, a single channel (e.g., channel 214a) may be utilized to route the plurality of conductors 216a, 216b, 216c to the electrodes 210a, 210b, 210c. In this embodiment, the conductors 216a, 216b, 216c include an insulating sheath to prevent short-circuiting. In another embodiment, the channels 214a, 214b, 214c house one or more conductors 216a, 216b, 216c, respectively. The conductors 216a, 216b, 216c may be encased in an insulating sheath (not shown) and are coupled to the electrodes 210a, 210b, 210c, respectively. The channels 214a, 214b, 214c are disposed apart (e.g., radially) within the core 202 and extend up to the respective electrodes 210a, 210b, 210c. The dielectric structure of the core 202 obviates the need for providing an insulating sheath about the conductors 216a, 216b, 216c since the conductors 216a, 216b, 216c are separated by the core 202 and only contact the corresponding electrodes 210a, 210b, 210c.

In one embodiment, the channels 214a, 214b, 214c may be disposed on an outer surface of the core 202. As shown in Fig. 7, the core 202 may include a sheath 217 disposed over the body portion 213 to insulate the conductors 216a, 216b, 216c. The sheath 217 includes openings 218a, 218b, 218c to provide for coupling of the conductors 216a, 216b, 216c to the electrodes 210a, 210b, 210c.

Each of the electrodes 210a, 210b, 210c is connected to each of a plurality of phase-shifting circuits 29a, 29b, 29c, etc. The output voltages from these phase-shifting circuits 29a, 29b, 29c have substantially the same amplitudes, however, the phases of the voltages are shifted relative to each other. In one embodiment, each of electrodes 210a, 210b, 210c forms its own group, therefore the voltages supplied thereto are also shifted by 120° based on formula (1). In use, as each of the electrodes 210a, 210b, 210c is energized by the phase-shifting circuits 29a, 29b, 29c, the remaining two electrodes 210a, 210b, 210c that are not energized act as a return electrode. In other words, the electrodes 210a, 210b, 210c shift roles as active and return electrodes due to the phase-shifted application of voltage. In another embodiment, two of the electrodes (e.g., electrodes 210a and 210c) form one group and the remaining electrode (e.g., electrode 210b) forms another group, hence, the voltages supplied to the two groups are shifted by 180°.

Fig. 8 shows another embodiment of an electrosurgical electrode assembly 300 according to an embodiment of the present disclosure that is substantially similar to the electrode assembly 200. The electrode assembly 300 includes a core 302 having an elongated cylindrical body portion 303. The body portion 303 includes a proximal end 304 coupled to the housing 6 (Fig. 1) and a distal end 306.

The body portion 303 also includes a lumen 330 defined therethrough and the electrode assembly 300 includes an outer tube 332 and an inner tube 334 disposed therein. The outer and inner tubes 332 and 334 are concentrically-disposed relative to each other and are coupled to the coolant system 15 allowing the coolant system 15 to circulate the coolant fluid 19 therethrough. The outer tube 332 includes a tip 308 having a tapered portion terminating in a sharp tip to allow for insertion into tissue with minimal resistance. In those cases where the energy applicator is inserted into a pre-existing opening, the tip 308 may be rounded or flat. In another embodiment the lumen 330 may not pass through the entire body portion 303 and may terminate at the distal end 306 of the core 302, in which case the tip 308 may be formed by the core 302 and/or an electrode 310a as discussed above.

The electrode assembly 300 also includes three or more electrodes 310a, 310b, 310c. The electrodes 310a, 310b, 310c have a substantially cylindrical tubular shape and are disposed about the core 302. The electrode 310a may be tapered to provide continuity for the tip 308. The electrodes 310a, 310b, 310c are separated by dielectric spacers 312a and 312b that are integrally formed with the core 302.

The core 302 includes a channel 314 defined therein for routing the plurality of conductors 316a, 316b, 316c to the electrodes 310a, 310b, 310c. In this embodiment, the conductors 316a, 316b, 316c include an insulating sheath to prevent short-circuiting and are coupled to the electrodes 310a, 310b, 310c, respectively. Each of the electrodes 310a, 310b, 310c is connected to each of a plurality of phase-shifting circuits 29a, 29b, 29c, etc. as discussed above with respect to the electrode assembly 200.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-

1. An electrosurgical instrument, comprising:

> a core including an elongated body portion;
> a plurality of electrodes having a tubular shape and disposed about the core; and
> at least one dielectric spacer disposed between each of the plurality of the electrodes, wherein the plurality of the electrodes is coupled to an electrosurgical generator configured to supply phased electrosurgical voltage to the plurality of electrodes to generate a potential difference between at least two of the plurality of the electrodes.

2. An electrosurgical system, comprising:

> an electrosurgical instrument including:

>> a core including an elongated body portion;
>> a plurality of electrodes having a tubular shape and disposed about the core; and
>> at least one dielectric spacer disposed between each of the plurality of the electrodes; and

> an electrosurgical generator coupled to each of the plurality of the electrodes and configured to supply phased electrosurgical voltage to the plurality of electrodes to generate a potential difference between at least two of the plurality of the electrodes.

3. An electrosurgical system according to paragraph 2, further comprising:

> a coolant system including:

>> a supply tank configured to store a coolant; and
>> a supply pump coupled to the supply tank and to the electrosurgical instrument and configured to circulate a coolant therethrough.

4. An electrosurgical instrument, comprising:

> a core including an elongated body portion;
> a plurality of electrodes having a tubular shape and disposed about the core;
> at least one dielectric spacer disposed between each of the plurality of the electrodes; and
> a plurality of conductors each of which couples a respective one of the plurality of electrodes to an electrosurgical generator configured to supply phased electrosurgical voltage to at least one of the plurality of electrodes to generate a potential difference between at least two of the plurality of the electrodes.

5. An electrosurgical instrument according to any one of the preceding paragraphs, further comprising a plurality of conductors configured to couple each of the plurality of electrodes to the electrosurgical generator.

6. An electrosurgical instrument according to paragraph 5, wherein the core includes at least one channel defined therein and the plurality of conductors is disposed within the at least one channel.

7. An electrosurgical instrument according to paragraph 5, wherein the core includes a plurality of channels defined therein that are disposed radially apart and each of the plurality of conductors is disposed within each of the plurality of channels.

8. An electrosurgical instrument according to paragraph 6 or 7, wherein each of the plurality of conductors is formed from a spring member.

9. An electrosurgical instrument according to any one of the preceding paragraphs, wherein the core includes a tapered tip at a distal end thereof configured to penetrate tissue.

10. An electrosurgical instrument according to any one of the preceding paragraphs, wherein a distal most electrode of the plurality of electrodes includes a tapered tip at a distal end thereof configured to penetrate tissue.

11. An electrosurgical instrument according to any one of the preceding paragraphs, wherein the core includes a lumen defined therethrough.

12. An electrosurgical instrument according to paragraph 11, further comprising:

> an outer tube disposed within the lumen; and

> an inner tube disposed concentrically relative to the outer tube, wherein the outer tube and the inner tube are coupled to a coolant system configured to circulate a coolant therethrough.

**Claims**

1. An electrosurgical system (1), comprising:

an electrosurgical generator (20) configured to generate a phased electrosurgical voltage; and an electrosurgical instrument (100, 200), including:

a core (102, 202) having an elongated body portion (103, 203) and at least one channel (114a, 114b) defined therein; a plurality of electrodes (110a, b, c; 210 a, b, c) disposed about the core, each of the plurality of the electrodes coupled to the electrosurgical generator such that the phased electrosurgical voltage supplied to the plurality of electrodes generates a potential difference between at least two of the plurality of the electrodes; and a plurality of spring conductors (116a, 116b, 216a) disposed within the at least one channel and configured to couple each of the plurality of electrodes to the electrosurgical generator.

2. The electrosurgical system according to claim 1, wherein the core includes a tapered tip (108, 208) configured to penetrate tissue.

3. The electrosurgical system according to any one of the preceding claims, wherein a distal most electrode (310a) of the plurality of electrodes includes a tapered tip configured to penetrate tissue.

4. The electrosurgical system according to any one of the preceding claims, wherein electrosurgical instrument further includes at least one dielectric spacer (112, 212a, 212b) separating the plurality of electrodes.

5. The electrosurgical system according to claim 4, wherein the at least one dielectric spacer is formed integrally with the core.

6. The electrosurgical system according to any one of the preceding claims, wherein the at least one channel (114a, 114b) is disposed on an outer surface of the core.

7. The electrosurgical system according to any one of the preceding claims, wherein the electrosurgical instrument further includes a sheath (117) disposed over the elongated body portion of the core.

8. The electrosurgical system according to claim 7, wherein the sheath ( 117) includes openings (118a, 118b) to provide for coupling of the conductors to the electrodes.

9. The electrosurgical system according to claim 8, wherein each conductor extends through a respective one of the openings.

10. The electrosurgical system according to any one of the preceding claims, further comprising:

a coolant system (15) including:

a supply tank (18) configured to store a coolant (19); and a supply pump (16) coupled to the supply tank and to the electrosurgical instrument and configured to circulate a coolant therethrough.

11. The electrosurgical system according to any one of the preceding claims, wherein the core includes a lumen (330) defined therethrough.

12. The electrosurgical system according to claim 11, wherein the electrosurgical instrument further includes:

an outer tube (332) disposed within the lumen; and an inner tube (334) disposed concentrically relative to the outer tube, wherein the outer tube and the inner tube are coupled to the coolant system.

**FIG. 1**

EP 3 053 537 A1

FIG. 2

FIG. 3

FIG. 4

FIG. 5

FIG. 6

FIG. 7

FIG. 8

Europäisches
Patentamt

European
Patent Office

Office européen
des brevets

# EUROPEAN SEARCH REPORT

**Application Number**

EP 16 15 6267

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/128643 A1 (SIMPSON JOHN A [US] ET AL) 12 September 2002 (2002-09-12) | 1-5,8,9 | INV. A61B18/14 |
| Y | * paragraphs [0064], [0067], [0059], [0135]; figure 1 * | 6,7, 10-12 | ADD. A61B18/12 |
| Y | US 5 935 159 A (CROSS JR THOMAS E [US] ET AL) 10 August 1999 (1999-08-10) * figures 2,3 * | 6,7 | A61B18/00 |
| Y | US 5 951 546 A (LORENTZEN TORBEN [DK]) 14 September 1999 (1999-09-14) * figures 2a,3 * | 10-12 | |
| A | US 2009/118728 A1 (SCIMED LIFE SYSTEMS INC [US]; OSTROVSKY ISAAC [US]; MCINTYRE JON T [US]) 7 May 2009 (2009-05-07) * the whole document * | 1-12 | |

TECHNICAL FIELDS
SEARCHED      (IPC)

A61B

The present search report has been drawn up for all claims

| Place of search | Date of completion of the search | Examiner |
|---|---|---|
| Munich | 29 June 2016 | Monogyiou, Efstratia |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

**EP 3 053 537 A1**

## ANNEX TO THE EUROPEAN SEARCH REPORT
## ON EUROPEAN PATENT APPLICATION NO.

EP 16 15 6267

29-06-2016

| Patent document cited in search report | Publication date | Patent family member(s) | Publication date |
|---|---|---|---|
| US 2002128643 A1 | 12-09-2002 | NONE | |
| US 5935159 A | 10-08-1999 | AU 5695298 A<br>EP 0971767 A2<br>JP 2002501402 A<br>US 5935159 A<br>WO 9829055 A2 | 31-07-1998<br>19-01-2000<br>15-01-2002<br>10-08-1999<br>09-07-1998 |
| US 5951546 A | 14-09-1999 | AU 4252596 A<br>EP 0797408 A2<br>EP 2070486 A1<br>EP 2314244 A1<br>US 5951546 A<br>WO 9618349 A2 | 03-07-1996<br>01-10-1997<br>17-06-2009<br>27-04-2011<br>14-09-1999<br>20-06-1996 |
| US 2009118728 A1 | 07-05-2009 | EP 1684655 A2<br>US 2005107781 A1<br>US 2009118728 A1<br>US 2012220995 A1<br>WO 2005048862 A2 | 02-08-2006<br>19-05-2005<br>07-05-2009<br>30-08-2012<br>02-06-2005 |